# EUROPEAN PATENT APPLICATION

(11) **EP 4 516 336 A1**
(43) Date of publication of application: **05.03.2025**
(21) Application number: 23306429.4
(22) Date of filing: 29.08.2023
(51) Int. Cl.: A61M 5/00

(54) **DEVICE AND PACKAGING SYSTEM FOR TRANSPORTING MEDICAL DEVICES**

(71) Applicant: Becton Dickinson France, 38800 Le Pont de Claix (FR)
(72) Inventor: REMPFER, Simon, 38260 Saint-Hilaire-de-la-Côte (FR); EYMERY, Anaïs, 38450 Saint-Georges-de-Commiers (FR); LAVIGNE, Ferdinand, 38170 Seyssinet Pariset (FR); PINEDA, Florian, 38000 Grenoble (FR)
(74) Representative: Regimbeau

(57) **Abstract**

A device for receiving medical containers or components of medical containers comprises a plate having an upper surface, a lower surface, and a sidewall extending therebetween. The sidewall is connected to the upper surface at an upper edge and the sidewall is connected to the lower surface at a lower edge. At least a portion of the upper edge and/or at least a portion of the lower edge is a curved surface. Methods of forming the device include injection molding the plate.

## Description

### TECHNICAL FIELD

The present disclosure relates generally to a device for transporting medical containers, such as syringes, and components thereof, such as stoppers, and an associated container in which the plate is received.

### BACKGROUND

Medical containers, such as pre-fillable or prefilled syringes, vials, and ampoules, and components for use with these containers, such as stoppers, may be transported from one site to another site. For instance, medical containers may be transported from a manufacturing site to a site at which the medical containers may be filled with a medicinal fluid. By way of further example, the medical containers may be manufactured and filled at the same site and subsequently transported to a storage site in a sterile packaging. During transportation, the medical containers may be supported by a holding device, which may be referred to as a nest. One or more holding devices may be disposed within a packaging having an opening allowing for placement of the holding devices therein and subsequently sealed by a sealing cover. The container may be referred to as a tub.

External dimensions of the holding devices being slightly smaller than inner dimension of the packaging for ease of the assembly, the holding device may be susceptible to moving relative to the packaging during transportation. As a result of such movement during long-distance transport, the holding device rubs against an inner surface of the packaging and may wear away material of the packaging or of the holding device so as to create particles of the packaging material and/or the holding device material. Because the packaging is sealed by the sealing cover, the particles remain inside the packaging throughout the remainder of transportation and storage. Such particles may be dispersed within the packaging and thus disposed within or on the medical containers stored inside the packaging. When the medical containers arrive at the destination, the medical containers are intended to be in a ready-to-use condition. By way of non-limiting example, when the medical containers are pre-fillable syringes, the syringes are intended to be in a clean and sterile condition upon arrival at the filling site so that the syringes may be filled with medicinal fluid. If the particles of the packaging material and/or of the holding device material are dispersed within or on the syringes, the syringes are not in a ready-to-use condition and must be cleaned prior to filling in order to avoid contamination of the medicinal fluid with which the medical containers will be filled. Accordingly, there is a need for a packaging solution that would inhibit or altogether prevent the creation of particles within the packaging.

### BRIEF SUMMARY

An objective of the present disclosure is to provide a device for receiving medical containers or components of medical containers solving the above-mentioned drawbacks. The device comprises:
a plate having an upper surface, a lower surface, and a sidewall extending between the upper surface and the lower surface, the plate defining a plurality of openings extending therethrough from the upper surface to the lower surface, each opening configured to retain a medical container or component of a medical container therein,
wherein the sidewall is connected to the upper surface at an upper edge and the sidewall is connected to the lower surface at a lower edge, the upper edge, the lower edge, and the sidewall defining a periphery of the plate,
wherein at least a portion of the upper edge and/or at least a portion of the lower edge is a curved surface.

Certain preferred but non-limiting features of the device described above are the following, taken individually or in combination:
the portion of the upper edge and the portion of the lower edge is a curved surface;
a radius of curvature of the curved surface is in a range from 0.03 mm to 1 mm, preferably about 0.3 mm;
the plate comprises a plurality of projections, each of the plurality of projections respectively defines the portion of the upper surface, the lower surface, the sidewall, the upper edge, and the lower edge of the plate, a minority of the periphery of the plate is defined by the plurality of projections;
the upper edge and the lower edge defining a majority of the periphery of the plate is a straight edge.

Another object of the present disclosure is a packaging system comprising:
the device as described above; and
a tub having a plurality of sidewalls having interior surfaces defining a space configured to receive the plate therein,
wherein, when the device is disposed in the tub, the curved surface of the upper edge and/or the lower edge contacts the interior surface of the tub.

Another object of the disclosure is a method of forming the device as described above. The method comprises:
injecting a polymeric material of the plate into a mold, the mold comprises at least one movable part and a fixed part,
wherein the at least one moveable part and the fixed part collectively define a mold cavity to form the plate,
wherein the at least one movable part and/or the fixed part has a curved surface configured to form the curved surface of the portion of the upper edge and/or the portion of the lower edge defined by each of the plurality of projections.

Certain preferred but non-limiting features of the method described above are the following, taken individually or in combination:
the fixed part is shaped to form the portion of the upper edge, the lower edge, the sidewall, and the lower surface defined by each of the plurality of projections,
the at least one movable part is shaped to form the portion of the upper surface defined by each of the plurality of projections of the plate, and
the fixed part includes curved surfaces configured to form curved surfaces at the upper edge and the lower edge defined by each of the plurality of projections;
a parting line of the fixed part and the at least one movable part is coextensive with the upper surface of the plate;
the fixed part is shaped to form the portion of the upper surface and the upper edge defined by the plurality of projections,
the at least one movable part is shaped to form the portion of the lower edge, the sidewall, and the lower surface of the plate defined by the plurality of projections, and
the at least one movable part includes curved surfaces configured to form the curved surface of the portion of the lower edge defined by the plurality of projections;
a parting line of the fixed part and the at least one movable part is coextensive with the upper surface of the plate;
the fixed part is shaped to form the portion of the upper surface, the upper edge and the sidewall defined by each of the plurality of projections,
the at least one movable part is shaped to form the portion of the lower surface, the lower edge and a portion of the sidewall defined by each of the plurality of projections, and
each of the fixed part and the at least one movable part includes curved surfaces to form the curved surface of the upper edge and the lower edge defined by each of the plurality of projections;
a parting line of the fixed part and the at least one movable part is located along the sidewall between the upper surface and the lower surface of the plate;
the at least one movable part comprises a slider and a movable part (202),
the slider is shaped to form the portion of the upper edge, the sidewall, and the lower edge defined by each of the plurality of projections, and
the slider includes curved surfaces to form the curved surface of the upper edge and the lower edge defined by each of the plurality of projections;
a parting line of the slider and the movable part is located along the upper surface of the plate, and a parting line of the slider and the fixed part is located along the lower surface of the plate.

### BRIEF DESCRIPTION OF THE DRAWINGS

The illustrations presented herein are not meant to be actual views of any particular component, device, or system, but are merely idealized representations which are employed to describe embodiments of the present invention. Other features, goals, and advantages of the present invention will appear more clearly upon reading of the detailed description that follows and with references to drawings provided by way of non-limiting examples wherein:
FIGS. 1 and 2 are views of a plate for a packaging system;
FIG. 3 is a view of a tub for a packaging system;
FIG. 4 is a top view of a packaging system;
FIGS. 5A and 5B illustrate a mold for the plate, respectively during injection of a polymeric material and during ejection of the plate from the mold, according to a first embodiment;
FIGS. 6A and 6B illustrate a mold for the plate, respectively during injection of a polymeric material and during ejection of the plate from the mold, according to a second embodiment;
FIGS. 7A and 7B illustrate a mold for the plate, respectively during injection of a polymeric material and during ejection of the plate from the mold, according to a third embodiment;
FIGS. 8A and 8B illustrate a mold for the plate, respectively during injection of a polymeric material and during ejection of the plate from the mold, according to a second embodiment.

### DETAILED DESCRIPTION

As used herein, relational terms, such as "first," "second," "top," "bottom," "upper," "lower," "over," "under," "on," "interior," "exterior," and the like, are used for clarity and convenience in understanding the disclosure and accompanying drawings and do not connote or depend on any specific preference, orientation, or order, except where the context clearly indicates otherwise.

As used herein, the term "and/or" includes any and all combinations of one or more of the associated listed items.

As used herein, the term "configured" refers to a size, shape, material composition, material distribution, orientation, and arrangement of one or more of at least one structure and at least one apparatus facilitating operation of one or more of the structure and the apparatus in a pre-determined way.

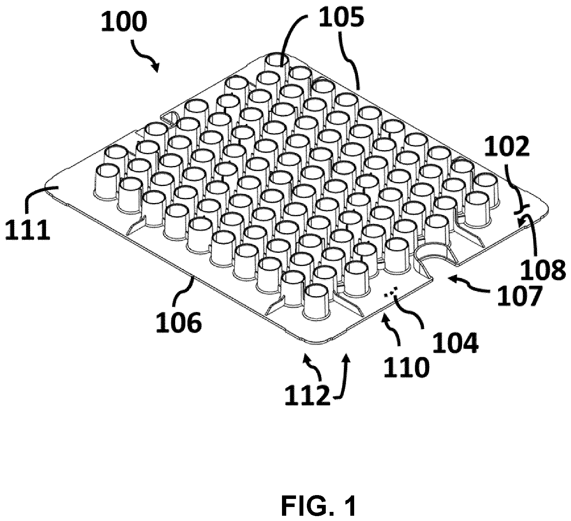

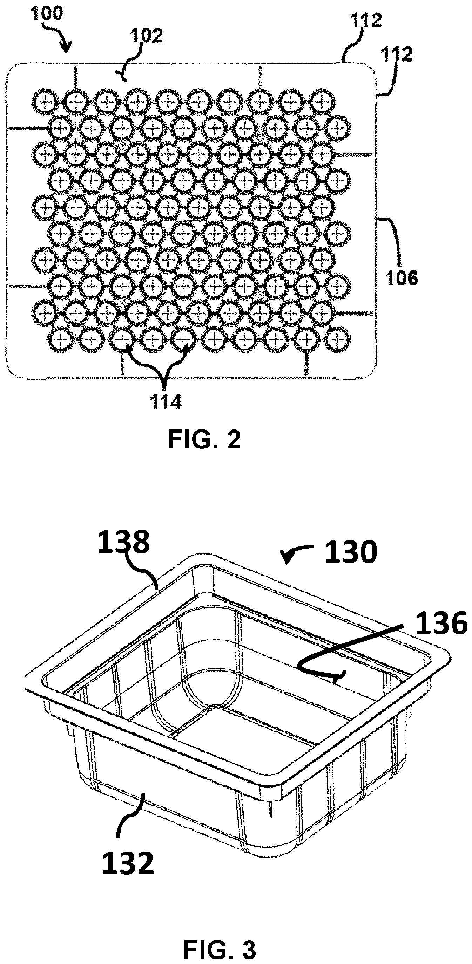

FIGS. 1 and 2 illustrate a device configured to support a plurality of medical containers or a plurality of components of a medical container therein. The device may be referred to in the art generally as a nest. The medical containers may be vials, syringes, such as pre-fillable or prefilled syringes, and the like or components of the medical container may be stoppers, syringe gaskets, and the like. The medical containers are configured, after filling, to contain a medical fluid therein.

The device comprises a plate 100 having an upper surface 102, a lower surface 104, and a sidewall 106 extending between the upper surface 102 and the lower surface 104.

The sidewall 106 defines a perimeter of the plate 100. Generally, the sidewall 106 comprises four edges connected by corners 111, which may be square or rounded. In some embodiments, indentations 107 may be formed in edges of the plate 100. Such indentations 107 are configured to be gripped by a person or a machine to facilitate insertion and removal the plate 100 from a tub 130.

The sidewall 106 is connected to the upper surface 102 at an upper edge 108, and the sidewall 106 is connected to the lower surface 104 at a lower edge 110. The upper edge 108, the lower edge 110, and the sidewall 106 collectively define a periphery of the plate 100.

In some embodiments, the plate 100 may comprise a plurality of projections 112. The projections 112 are those portions of the plate 100 having the greatest lateral dimensions (e.g., a width or length).

Each of the projections 112 respectively defines a portion of the periphery of the plate 100. The projections 112 are each defined by a portion of the upper surface 102, the lower surface 104, the sidewall 106, the upper edge 108, and the lower edge 110 of the plate 100.

The plurality of projections 112 define a minority of the periphery of the plate 100, and a remainder of the periphery of the plate 100 is recessed relative to the portion of the sidewall 106 defined by the projections 112. When the plate 100 is disposed in the tub 130, a space is provided between the remainder of the sidewall 106 and an interior surface 136 of the tub 130.

In other embodiments (not illustrated), the plate 100 may lack projections.

A longitudinal axis of the plate 102 extends perpendicular to (e.g., normal to) the upper surface 102. Apart from features such as chimneys 105, the upper surface 102 of the plate 100 is substantially planar. The lower surface 104 may also be substantially planar.

The plate 100 defines a plurality of openings 114 extending therethrough from the upper surface 102 to the lower surface 104. Each opening 114 is configured to retain a medical container or component of a medical container therein.

The plate 100 may also comprise a plurality of chimneys 105 that protrude axially from the upper surface 102. Each chimney 105 extends at least partially circumferentially about a corresponding opening 114. Each chimney 105 may be a generally cylindrical structure. Each chimney 105 defines an opening within which a medical container may be retained. Each chimney 105 may have a centrally extending longitudinal axis that is parallel with the longitudinal axis of the plate 102. Each chimney 105 may be integrally formed with the plate 100.

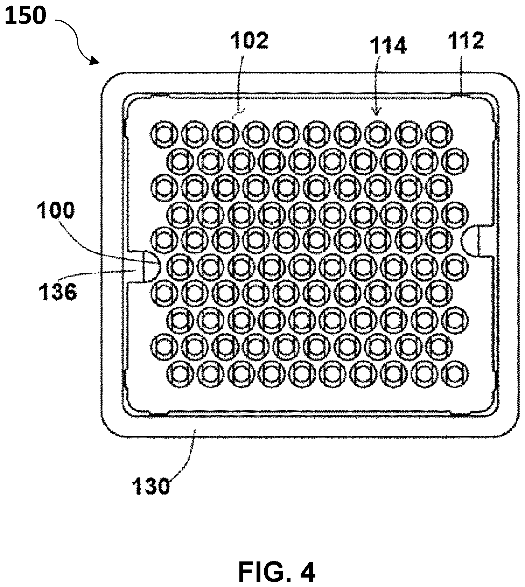

FIG. 3 illustrates a tub. As shown in FIG. 4, the packaging system 150 comprises at least one plate 100 disposed in the tub 130 for receiving and transporting a plurality of medical containers or components of medical containers.

The tub 130 comprises a plurality of sidewalls 132. The sidewalls 132 may extend from a bottom wall that defines a bottom of the tub 130 to a flange 138 that defines a top of the tub 130. The interior of the tub 130 is hollow and is defined by an interior surface of the bottom wall and an interior surface 136 of the sidewalls 132.

The tub 130 is open such that the plate(s) 100 may be inserted therein. The flange 138 may be provided at an end of the sidewalls 132 opposite to which the bottom wall is located. After one or more plates 100 having medical containers or medical container components therein are disposed in the tub 130, a sealing element may be coupled to a surface of the flange 138 to close the opening of the tub 130 and allow for sterilization of the packaging system.

When the plate 100 is disposed in the tub 130, at least a portion of the sidewall 106 of the plate 100 contacts the interior surface 136 of the tub 130. Further, at least a portion of the upper edge 108 and/or the lower edge 110 contacts the interior surface 136 of the sidewalls 132 of the tub 130.

In embodiments in which the plate 100 comprises projections 112, the sidewall 106, the upper edge 108, and/or the lower edge 110 defined by the projections contacts the interior surface 136 of the tub 130 while a remainder of the sidewall 106, the upper edge 108, and lower edge 110 are separated from (e.g., not in contact) with the interior surface 136 of the sidewalls of the tub 130.

In some embodiments, the sidewalls 132 of the tub 130 are disposed at an obtuse angle relative to the bottom wall such that the cross-sectional area of the tub 130 decreases between the top and the bottom of the tub 130. In this case, only the lower edge 110 and a portion of the sidewall 106 of the plate 100 may contact the interior surface 136 of the sidewalls of the tub 130.

In other embodiments, the sidewalls 132 of the tub 130 are disposed at a substantially right angle relative to the bottom wall such that the cross-sectional area defined by interior surfaces 136 of the tub 130 is substantially constant between the top and the bottom of the tub 130. In this case, each of the sidewall 106, the upper edge 108 and the lower edge 110 of the plate 100 may contact the interior surface 136 of the sidewalls of the tub 130.

The lower edge 110 and/or the upper edge 108 of the plate 100 comprises a curved, or rounded, surface. In some embodiments, the portion of the upper edge 108 and/or the portion of the lower edge 110 defined by each of the plurality of projections 112 is a curved surface. In such embodiments, a remainder of the upper edge 108 and/or the lower edge 110 defining the majority of the periphery of the plate 100 may be a straight edge. In other embodiments, especially of the plate does not include projections, the entirety of the upper edge 108 and/or the lower edge 110 may be a curved surface.

The presence of the curved surface along the edge of the plate 100 intended to contact interior surfaces of the tub 130 once disposed therein prevents the creation of particles of the materials of the plate 100 or the tub 130 when the plate 100 having the curved upper and/or lower edge rubs against the interior surface 136 of the tub 130 transportation.

A radius of curvature of the curved surface is in a range from 0.03 mm to 1 mm, preferably about 0.3 mm.

The present disclosure also relates to a method of forming the plate 100 such that the upper edge 108 and/or the lower edge 110 comprises a curved surface as previously described herein.

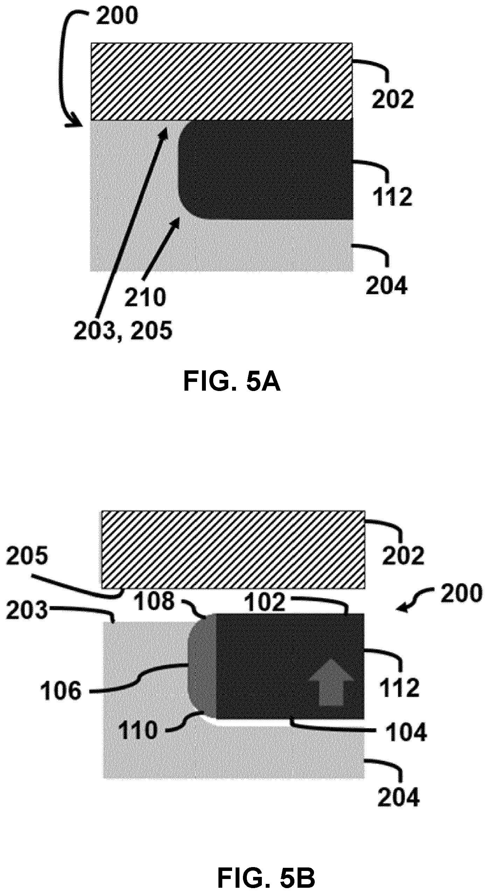

The plate 100 may be formed in an injection molding process. In the method, a polymeric material of the plate 100 is injected into a mold 200. Partial views of molds 200 usable in an injection molding process are shown in FIGS. 5A-8B. The portions of the molds 200 illustrated are cross sections of those portions of the mold 200 having cavities configured to form the projections 112.

The mold 200 comprises at least one movable part 202 and a fixed part 204. The moveable part 202 and the fixed part 204 collectively define a mold cavity 210 to form the plate 100. The movable part 202 and/or the fixed part 204 has a curved surface configured to form the curved surface of the portion of the upper edge 108 and/or the portion of the lower edge 110 defined by each of the plurality of projections 112.

In an embodiment illustrated in FIGS. 5A and 5B, both the upper edge 108 and lower edge 110 have curved surfaces. The fixed part 204 is shaped to form the portion of the upper edge 108, the lower edge 110, the sidewall 106, and the lower surface 104 defined by each of the plurality of projections 112. The movable part 202 is shaped to form the portion of the upper surface 106 defined by each of the plurality of projections 112. The fixed part 204 includes curved surfaces configured to form curved surfaces at the upper edge 108 and the lower edge 110 defined by each of the plurality of projections 112.

A surface 203 of the movable part 202 and a surface 205 of the fixed part 204 come together for the injection portion of the process and meet at a location referred to in the art as the parting line.

In the embodiment of FIGS. 5A and 5B, the parting line is co-located with the upper surface 106 of the plate 100.

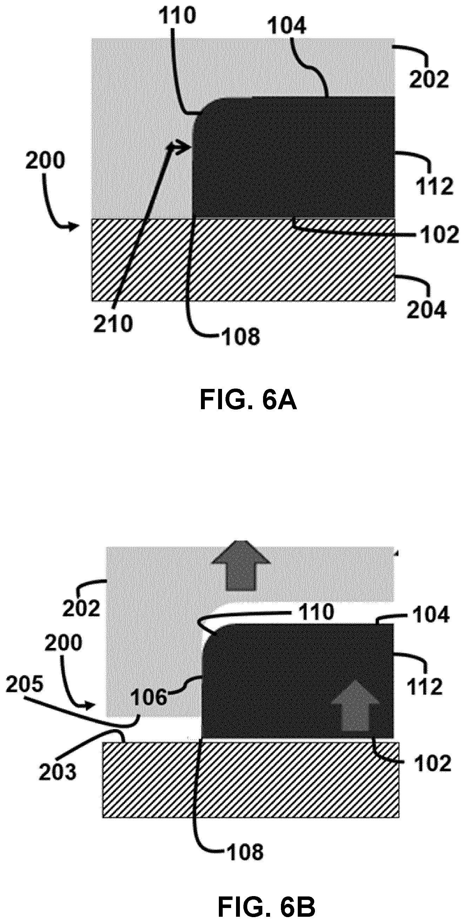

In an embodiment illustrated in FIGS. 6A and 6B, only the lower edge 110 has a curved surface. The fixed part 204 is shaped to form the portion of the upper surface 102 and the upper edge 108 defined by the plurality of projections 112, and the at least one movable part 202 is shaped to form the portion of the lower edge 110, the sidewall 106, and the lower surface 104 of the plate 100 defined by the plurality of projections 112. The at least one movable part 202 includes curved surfaces configured to form the curved surface of the portion of the lower edge 110 defined by the plurality of projections 112. In this embodiment, the upper edge 108 defined by the plurality of projections 112 comprises a straight edge. A parting line of the fixed part 204 and the at least one movable part 202 is coextensive with the upper surface 102 of the plate 100.

Conversely, if only the upper edge 108 has a curved surface and the lower edge 110 has a straight edge, the mold illustrated in FIGS. 6A and 6B can be used with the fixed part 204 shaped to form the portion of the lower surface and the lower edge defined by the plurality of projections and the movable part 202 including curved surfaces configured to form the curved surface of the upper edge defined by the plurality of projections 112.

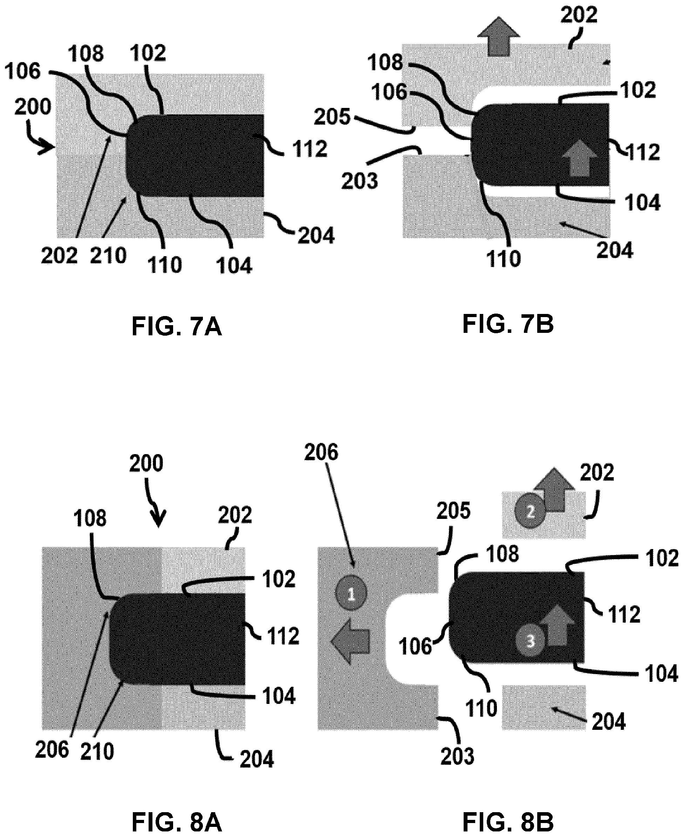

In an embodiment illustrated in FIGS. 7A and 7B, the fixed part 204 is shaped to form the portion of the upper surface 102, the upper edge 108 and the sidewall 106 defined by each of the plurality of projections 112, and the at least one movable part 202 is shaped to form the portion of the lower surface 104, the lower edge 110 and a portion of the sidewall 106 defined by each of the plurality of projections 112. Each of the fixed part 204 and the at least one movable part 202 includes curved surfaces to form the curved surface of the upper edge 108 and the lower edge 110 defined by each of the plurality of projections 112. The parting line 203 of the fixed part 204 and the at least one movable part 202 is located along the sidewall 106 between the upper surface 102 and the lower surface 104 of the plate 100.

In an embodiment illustrated in FIGS. 8A and 8B, the movable part 202 includes two distinct mold pieces. A movable part 202 comprising a cavity and a slider 206 comprising a cavity. The slider 206 is shaped to form the portion of the upper edge 108, the sidewall 106, and the lower edge 110 defined by each of the plurality of projections 112. The slider 206 includes curved surfaces to form the curved surface of the upper edge 108 and the lower edge 110 defined by each of the plurality of projections 112. A parting line (205) between the slider 206 and the movable part 202 is located along the upper surface 102 of the plate 100, and a parting line (203) of the slider 206 and the fixed part 204 is located along the lower surface 104 of the plate 100.

In injection molding, parts are subject to flash when molten plastic flows out of the mold 200 along the parting line and solidifies. If such flash is generated along a surface of the projections 112 that contacts the interior surface of the tub 130, particles of the tub 130 or plate 100 may be generated due to rubbing of the flash against the interior surface of the tub 130. The method may comprise a step of removing this flash by mechanical methods, such as by cutting, breaking, grinding, or tumbling, or by chemical methods, such as etching, before the medical containers are disposed in the openings 114 of the plate 100 and/or before the plate 100 is disposed in the tub 130. Locating the parting line along the upper surface 102 and/or lower surface 104 of the projections 112 as described herein, which surfaces do not generally contact an interior surface of the sidewalls 132 of the tub, reduces the potential for particles to be generated by any flash material unintentionally remaining after deflashing.

While the present disclosure has been described herein with respect to certain illustrated embodiments, those of ordinary skill in the art will recognize and appreciate that it is not so limited. Rather, many additions, deletions, and modifications to the illustrated embodiments may be made without departing from the scope of the disclosure, including legal equivalents thereof. In addition, features from one embodiment may be combined with features of another embodiment while still being encompassed within the scope of the invention as contemplated by the inventors.

## Claims

1. A device for receiving medical containers or components of medical containers, comprising:
a plate (100) having an upper surface (102), a lower surface (104), and a sidewall (106) extending between the upper surface (102) and the lower surface (104), the plate (100) defining a plurality of openings (114) extending therethrough from the upper surface (102) to the lower surface (104), each opening (114) configured to retain a medical container or component of a medical container therein,
wherein the sidewall (106) is connected to the upper surface (102) at an upper edge (108) and the sidewall (106) is connected to the lower surface (104) at a lower edge (110), the upper edge (108), the lower edge (110), and the sidewall (106) defining a periphery of the plate (100),
wherein at least a portion of the upper edge (108) and/or at least a portion of the lower edge (110) is a curved surface.

2. The device of claim 1, wherein the portion of the upper edge (108) and the portion of the lower edge (110) is a curved surface.

3. The device of claim 1 or 2, wherein a radius of curvature of the curved surface is in a range from 0.03 mm to 1 mm, preferably about 0.3 mm.

4. The device of any of claims 1-3, wherein the plate (100) comprises a plurality of projections (112), each of the plurality of projections (112) respectively defines the portion of the upper surface (102), the lower surface (104), the sidewall (106), the upper edge (108), and the lower edge (110) of the plate (100), a minority of the periphery of the plate (100) is defined by the plurality of projections (112).

5. The device of claim 4, wherein the upper edge (108) and the lower edge (110) defining a majority of the periphery of the plate (100) is a straight edge.

6. A packaging system (150), comprising:
the device of any of claims 1-5; and
a tub (130) having a plurality of sidewalls (132) having interior surfaces (136) defining a space configured to receive the plate (100) therein,
wherein, when the device is disposed in the tub (130), the curved surface of the upper edge (108) and/or the lower edge (110) contacts the interior surface (136) of the tub (130).

7. A method of forming the device according to any of claims 1-5, comprising injecting a polymeric material of the plate (100) into a mold (200), the mold comprises at least one movable part (202) and a fixed part (204), wherein the at least one moveable part (202) and the fixed part (204) collectively define a mold cavity (210) to form the plate (100), wherein the at least one movable part (202) and/or the fixed part (204) has a curved surface configured to form the curved surface of the portion of the upper edge (108) and/or the portion of the lower edge (110) defined by each of the plurality of projections (112).

8. The method of claim 7, wherein:
the fixed part (204) is shaped to form the portion of the upper edge (108), the lower edge (110), the sidewall (106), and the lower surface (104) defined by each of the plurality of projections (112),
the at least one movable part (202) is shaped to form the portion of the upper surface (106) defined by each of the plurality of projections of the plate (100), and
the fixed part (204) includes curved surfaces configured to form curved surfaces at the upper edge (108) and the lower edge (110) defined by each of the plurality of projections (112).

9. The method of claim 8, wherein a parting line (203, 205) of the fixed part (204) and the at least one movable part (202) is coextensive with the upper surface (106) of the plate (100).

10. The method of claim 7, wherein:
the fixed part (204) is shaped to form the portion of the upper surface (102) and the upper edge (108) defined by the plurality of projections (112),
the at least one movable part (202) is shaped to form the portion of the lower edge (110), the sidewall (106), and the lower surface (104) of the plate (100) defined by the plurality of projections (112), and
the at least one movable part (202) includes curved surfaces configured to form the curved surface of the portion of the lower edge (110) defined by the plurality of projections (112).

11. The method of claim 10, wherein a parting line of the fixed part (204) and the at least one movable part (202) is coextensive with the upper surface (102) of the plate (100).

12. The method of claim 7, wherein:
the fixed part (204) is shaped to form the portion of the upper surface (102), the upper edge (108) and the sidewall (106) defined by each of the plurality of projections (112),
the at least one movable part (202) is shaped to form the portion of the lower surface (104), the lower edge (110) and a portion of the sidewall (106) defined by each of the plurality of projections (112), and
each of the fixed part (204) and the at least one movable part (202) includes curved surfaces to form the curved surface of the upper edge (108) and the lower edge (110) defined by each of the plurality of projections (112).

13. The method of claim 12, wherein a parting line (203, 205) of the fixed part (204) and the at least one movable part (202) is located along the sidewall (106) between the upper surface (102) and the lower surface (104) of the plate (100).

14. The method of claim 7, wherein:
the at least one movable part (202) comprises a slider (206) and a movable part (202),
the slider (206) is shaped to form the portion of the upper edge (108), the sidewall (106), and the lower edge (110) defined by each of the plurality of projections (112), and
the slider (206) includes curved surfaces to form the curved surface of the upper edge (108) and the lower edge (110) defined by each of the plurality of projections (112).

15. The method of claim 14, wherein a parting line (205) of the slider (206) and the movable part (202) is located along the upper surface (102) of the plate (100), and wherein a parting line (203) of the slider (206) and the fixed part (204) is located along the lower surface (104) of the plate (100).
